# EUROPEAN PATENT SPECIFICATION

(11) **EP 4 074 325 B1**
(45) Date of publication and mention of the grant of the patent: **31.07.2024**
(21) Application number: 22167800.6
(22) Date of filing: 12.04.2022
(51) Int. Cl.: A61K 36/82, A61K 31/00, A61P 31/14

(54) **COMPOSITION FOR PREVENTING, INHIBITING OR TREATING INFECTION OF CORONAVIRUS**
ZUSAMMENSETZUNG ZUM VORBEUGEN, HEMMEN ODER BEHANDELN EINER INFEKTION MIT CORONAVIREN
COMPOSITION POUR PRÉVENIR, INHIBER OU TRAITER UNE INFECTION CORONAVIRALE

(30) Priority: 13.04.2021 KR 20210047945
(43) Date of publication of application: 19.10.2022
(73) Proprietor: Amorepacific Corporation, Seoul 04386 (KR)
(72) Inventor: CHOI, Hyunjung, 17074 Yongin-si, Gyeonggi-do (KR); KO, Jaeyoung, 17074 Yongin-si, Gyeonggi-do (KR); LEE, Yonghee, 17074 Yongin-si, Gyeonggi-do (KR); KIM, Hyoung June, 17074 Yongin-si, Gyeonggi-do (KR); PARK, Wonseok, 17074 Yongin-si, Gyeonggi-do (KR); BAEK, Heungsoo, 17074 Yongin-si, Gyeonggi-do (KR)
(74) Representative: Germain Maureau

(56) References cited:
- WO-A1-2020/235717
- KR-B1- 101 638 818
- CAO THAO QUYEN ET AL: "SARS-CoV-2 main protease inhibition by compounds isolated from Luffa cylindrica using molecular docking", BIOORGANIC & MEDICINAL CHEMISTRY LETTERS, ELSEVIER, AMSTERDAM, NL, vol. 40, 19 March 2021 (2021-03-19), XP086557656, ISSN: 0960-894X, [retrieved on 20210319], DOI: 10.1016/J.BMCL.2021.127972
- SHARMA ET AL: "Saponins: Extraction, bio-medicinal properties and way forward to anti-viral representatives - ScienceDirect", 19 February 2021 (2021-02-19), pages 1 - 33, XP055927544, Retrieved from the Internet <URL:https://www.sciencedirect.com/science/article/pii/S0278691521001083?via=ihub> [retrieved on 20220602]
- LUO PAN ET AL: "Pharmacological perspective: glycyrrhizin may be an efficacious therapeutic agent for COVID-19", INTERNATIONAL JOURNAL OF ANTIMICROBIAL AGENTS, ELSEVIER, AMSTERDAM, NL, vol. 55, no. 6, 24 April 2020 (2020-04-24), XP086159031, ISSN: 0924-8579, [retrieved on 20200424], DOI: 10.1016/J.IJANTIMICAG.2020.105995
- CUI CHUANJIAN ET AL: "Triterpenoid saponins from the genus Camellia : structures, biological activities, and molecular simulation for structure-activity relationship", FOOD & FUNCTION, vol. 9, no. 6, 1 January 2018 (2018-01-01), GB, pages 3069 - 3091, XP093060134, ISSN: 2042-6496, DOI: 10.1039/C8FO00755A

## Description

### [Technical Field]

The present specification relates to a composition for preventing, inhibiting or treating coronavirus infection.

### [Background Art]

Coronaviruses are enveloped, positive-sense single strand RNA viruses and have 25-32 kb of genome size, so they belong to a relatively large RNA virus. Coronaviruses have a unique flame shape because the spike proteins, which are club-shaped bumps, are embedded in a membrane.

After the first discovery from chickens in 1937, coronaviruses have been found in various birds and mammals such as bat, bird, cat, dog, cow, pig and mouse. Coronaviruses are divided into four groups (Alpha-, Beta-, Gamma- and Deltacoronavirus). Alphacoronavirus and Betacoronavirus groups primarily infect mammals, and Gammacoronavirus and Deltacoronavirus groups are discovered from birds. It has been known that coronaviruses cause various diseases such as gastrointestinal diseases and respiratory diseases.

Human coronaviruses, which infect human beings, were discovered, and SARS coronavirus (SARS-CoV), causing severe acute respiratory syndrome (SARS), was first discovered in 2003. In relation to SARS, according to a report of the World Health Organization (WHO), there were 8,273 patients and 775 deaths (fatality rate: about 10%) all over the world in 2002 and 2003. There were additional cases and deaths until 2004. In addition, the first identified case of infection by Middle East respiratory syndrome-coronavirus (MERS-CoV) occurred in Saudi Arabia in September 2012. And then, there were 808 cases and 313 deaths officially reported to the WHO by June 2014.

Recently, the world is threatened by the coronavirus disease-19 (COVID-19), a respiratory infectious disease that has been spreading since its first outbreak in Wuhan, China. The pathogen of COVID-19 is a new type of severe acute respiratory syndrome coronavirus 2 (SARS-Cov-2), transmitted when droplets (saliva) of an infected person penetrate the respiratory tract or the mucous membranes of the eyes, nose, and mouth.

As such, the coronaviruses have become a global problem, but an effective therapeutic agent and preventive vaccine have not been developed. Therefore, it is very urgent to develop a composition that effectively inhibits the infection of the coronavirus.

### [Disclosure]

### [Technical Problem]

The present invention is defined by the claims. The problem to be solved is how to prevent, inhibit or treat severe acute respiratory syndrome coronavirus-2 (SARS-CoV-2) infection.

### [Technical Solution]

In one embodiment the invention discloses: a camellia japonica saponin for use in preventing, inhibiting or treating coronavirus infection, wherein the camellia japonica saponin is extracted from oil press cake of seeds of camellia japonica, wherein the coronavirus is severe acute respiratory syndrome coronavirus-2 (SARS-CoV-2), wherein the camellia japonica saponin comprises camellia japonica saponin A1, camellia japonica saponin A2, camellia japonica saponin B 1, camellia japonica saponin B2, camellia japonica saponin C1, or a combination thereof.

In In an exemplary embodiment, the camellia saponin may be administered at a dosage of 60 to 150 mg/kg/day.

In an exemplary embodiment, the composition may be a composition for skin external application.

In an exemplary embodiment, the composition may be a food composition, a pharmaceutical composition or a cosmetic composition.

### [Advantageous Effects]

In one aspect, a composition according to the present disclosure can prevent, inhibit or treat infection by SARS-CoV-2.

In one aspect, a composition according to the present disclosure can eliminate the coronavirus remaining on the skin to block an infection mechanism of the coronavirus penetrating into a human body.

### [Brief Description of Drawings]

FIG. 1 is a graph showing the coronavirus infection inhibitory effect of Example 1 according to an embodiment of the present disclosure.
FIG. 2 is a graph showing the coronavirus infection inhibitory effect of Comparative Example 1 according to an embodiment of the present disclosure.
FIG. 3 is a graph showing the coronavirus infection inhibitory effect of Comparative Example 2 according to an embodiment of the present disclosure.

### [Detailed Description of the invention]

Hereinafter, the present disclosure is described in detail.

In one aspect, the present disclosure provides a composition for preventing, inhibiting or treating coronavirus infection comprising camellia saponin as an active ingredient.

In the present disclosure, camellia refers to *Camelliajaponica* which is an evergreen tree of the family Charysis, and is mainly distributed in East Asia including Korea. The oil is extracted from the seeds of the camellia to make camellia oil, and all parts of the plant such as roots, stems, leaves, flowers, etc. of the camellia may be used, and the entire plant (whole plant) may also be used.

In the present invention camellia saponin is the saponin derived from camelia japonica comprising camellia saponin A1, A2, B1, B2, C1 or combination thereof. In the present disclosure the saponins may be prepared by extracting the extract from the camellia using a solvent and removing impurities from the extract using an ion exchange resin, but is not limited thereto. The solvent may be water, alcohol, isopropanol, acetone, hexane, ethyl acetate, carbon dioxide, or a mixture of two or more thereof, but is not limited thereto. The alcohol may be a C₁-C₅ lower alcohol.

In one embodiment, the camellia saponin may be extracted from oil press cake of camellia.

In the present disclosure, the oil press cake may refer to a product remaining after squeezing oil and the like from seeds of plants. In the present disclosure, the oil press cake of camellia may refer to a product remaining after squeezing oil and the like from the seeds of camellia.

In one embodiment, the camellia saponin may be administered at a dosage of 60 to 150 mg/kg/day. If the dosage of camellia saponin is less than 60 mg/kg/day, it may be difficult to exhibit the effect according to the present invention, and if it exceeds 150 mg/kg/day, it may cause irritation to the skin. Specifically, the camellia saponin may be administered at a dose of 60 mg/kg/day or more, 61 mg/kg/day or more, 62 mg/kg/day or more, 63 mg/kg/day or more, 64 mg/kg/day or more, 65 mg/kg/day or more, 66 mg/kg/day or more, 67 mg/kg/day or more, 68 mg/kg/day or more, 69 mg/kg/day or more, 70 mg/kg/day or more, 71 mg/kg/day or more, 72 mg/kg/day or more, 73 mg/kg/day or more, 74 mg/kg/day or more, 75 mg/kg/day or more, 76 mg/kg/day or more, 77 mg/kg/day or more, 78 mg/kg/day or more, 79 mg/kg/day or more, 80 mg/kg/day or more, 81 mg/kg/day or more, 82 mg/kg/day or more, 83 mg/kg/day or more, 84 mg/kg/day or more, 85 mg/kg/day or more, or 150 mg/kg/day or less, 149 mg/kg/day or less, 148 mg/kg/day or less, 147 mg/kg/day or less, 146 mg/kg/day or less, 145 mg/kg/day or less, 144 mg/kg/day or less, 143 mg/kg/day or less, 142 mg/kg/day or less, 141 mg/kg/day or less, 140 mg/kg/day or less, 139 mg/kg/day or less, 138 mg/kg/day or less, 137 mg/kg/day or less, 136 mg/kg/day or less, 135 mg/kg/day or less, 134 mg/kg/day or less, 133 mg/kg/day or less, 132 mg/kg/day or less, 131 mg/kg/day or less or 130 mg/kg/day or less.

In one embodiment, the administration route of the composition is not limited, but may preferably be for skin external application.

A cosmetic composition is also described. The cosmetic composition may have a formulation such as a softening lotion, a astringent lotion, a nourishing lotion, a nourishing cream, a massage cream, an eye cream, an eye essence, an essence, a cleansing cream, a cleansing lotion, a cleansing foam, a cleansing water, a pack, a powder, a body lotion, a body cream, a body essence, a body cleaner, a hair dye, a shampoo, a conditioner, a hair fixative, a hair tonics, an ointment, a gel, a cream, a patch, a spray, a powder, and a skin adhesive type, but is not limited thereto.

In addition, in each formulation, the components other than the above essential components can be suitably selected and formulated by those skilled in the art without difficulty depending on the type or purpose of use of other external preparation.

The cosmetic composition may be provided in any formulation suitable for topical application. For example, it may be provided in the form of a solution, an emulsion obtained by dispersing an oil phase in an aqueous phase, an emulsion obtained by dispersing an aqueous phase in an oil phase, a suspension, a solid, a gel, a powder, a paste, a microneedle, a foam, or an aerosol composition. Compositions of such formulations can be prepared according to conventional methods in the art.

The cosmetic composition according to the present disclosure may further comprise functional additives and components included in general cosmetic compositions in addition to the compound of the present disclosure. The functional additive may comprise a component selected from the group consisting of a water-soluble vitamin, an oil-soluble vitamin, a high-molecular peptide, a high-molecular polysaccharide, a sphingolipid, and seaweed extract. The cosmetic composition according to the present specification may preferably comprise other ingredients that can give a synergistic effect to the main effect, within a range that does not impair the main effect. In addition, the cosmetic composition according to the present specification may further comprise a moisturizing agent, an emollient, a surfactant, a UV absorber, a preservative, a bactericide, an antioxidant, a pH adjuster, an organic and inorganic pigment, a fragrance, a cooling agent or an anti-perspiration agent. The blending amount of the component can be easily selected by those skilled in the art within the range that does not impair the purpose and effect of the present specification, and the blending amount may be 0.001 to 10% by weight, specifically 0.01 to 3% by weight, based on the total weight of the composition.

A food composition is also described. The formulation of the food composition is not particularly limited, and for example, it may be formulated as a tablet, granules, a pill, a powder, a liquid such as a drink, a caramel, a gel, a bar, a tea bag, and the like. In addition to the active ingredient, the food composition of each formulation can be combined with ingredients commonly used in the field selected by those skilled in the art without difficulty depending on the formulation or purpose of use, and when applied simultaneously with other raw materials, a synergistic effect may occur.

The food composition according to one embodiment may comprise various nutrients, a vitamin, a mineral (electrolyte), a flavoring agent such as synthetic and natural flavoring agents, a coloring agent, a thickener (cheese, chocolate, etc.), a pectic acid and salt thereof, an alginic acid and salt thereof, an organic acid, a protective colloidal thickening agent, a pH adjuster, a stabilizer, a preservative, a glycerin, an alcohol, a carbonation agent used in carbonated beverages, and the like. In addition, the food composition according to an embodiment may comprise a natural fruit juice and pulp for the production of a fruit juice beverage and a vegetable beverage. This component may be used independently or in combination. The proportion of this additive is not so important, but is generally included in the range of 0 to about 50 parts by weight per 100 parts by weight of the composition according to an embodiment.

In one embodiment, the composition may be a pharmaceutical composition. The pharmaceutical composition may be administered orally, parenterally, rectally, topically, transdermally, intravenously, intramuscularly, intraperitoneally, subcutaneously, and the like. The formulation for oral administration may be a tablet, a pill, soft and hard capsules, granules, a powder, fine granules, a liquid, an emulsion, or a pellet, but is not limited thereto.

The formulation for parenteral administration may be a liquid, a suspension, an emulsion, a gel, an injection, a drop, a suppository, a patch, or a spray, but is not limited thereto. The formulation can be easily prepared according to conventional methods in the art, and comprise a surfactant, an excipient, a wetting agent, an emulsification promoter, a suspending agent, a salt or buffer for control of osmotic pressure, a colorant, a spice, a stabilizer, an antiseptic, a preservative or other commercially available adjuvants.

Hereinafter, the present disclosure is described in more detail through examples and the like. These examples are only for illustrating the present disclosure, and it will be apparent to those of ordinary skill in the art that the scope of the present disclosure is not to be construed as being limited by these examples and is only limited by the wording of the claims.

### [Example 1] Preparation of camellia saponin

1 kg of oil press cake of camellia (Jeju Island) was milked at a low temperature to remove oil, and 8 kg of 70% ethanol was added. Extraction was performed at 60 °C for 8 hours using a warm extractor and filtering was performed by a filter press to obtain an extract of oil press cake of camellia. The extract of the oil press cake of the camellia was concentrated under a reduced pressure at 50 to 60 °C with a rotary vacuum concentrator until a solid content was about 50% of the concentrate, so that 200 g of the concentrate was obtained. The concentrate was diluted with purified water so as to have the solid content of 10%. After loading this solution on a glass tube column filled with 2L of ion exchange resin (HP-20), it was washed with 10L of purified water to remove impurities, sugars, and proteins, and then 10L of 30% ethanol was flowed. After that, 10 L of 50% ethanol was flowed, and the eluate was concentrated under a reduced pressure and freeze-dried to obtain 35 g of camellia saponin powder.

The thus obtained camellia saponin powder was dissolved in 70% ethanol, and the camellia saponin A1, A2, B1, B2 and C1 were sequentially separated by reverse-phase chromatography under the conditions of 0.1% TFA water/0.1% TFA acetonitrile and the content was analyzed, which is shown in Table 1.

**[Table 1]**

| | Retention time (min) | Area (%) |
|---|---|---|
| camellia saponin A1 | 22.03 | 2.0 |
| camellia saponin A2 | 18.82 | 4.4 |
| camellia saponin B1 | 16.12 | 47.7 |
| camellia saponin B2 | 13.96 | 25.2 |
| camellia saponin C1 | 13.31 | 6.1 |

### [Comparative Example 1]

Propolis was obtained from Hyundai Bioland and used as Comparative Example 1.

### [Comparative Example 2]

1-Deoxynojirimycin was obtained from Sigma-Aldrich and used as Comparative Example 2.

### [Experimental example 1] Evaluation of coronavirus infection inhibition efficacy and cytotoxicity

### 1. Coronaviruses and cell lines

Severe acute respiratory syndrome virus-2 (SARS-CoV-2) was provided by the Korea Centers for Disease Control and Prevention (KCDC), and Vero cells ((ATCC-CCL81) used in the experiment were obtained from ATCC company.

### 2. Reagent

A primary antibody, protein-specific anti-SARS-CoV-2 N was obtained from Sino Biological, and secondary antibodies, Alexa Fluor 488 goat anti-rabbit IgG and Hoechst 33342, were obtained from Molecular Probes.

### 3. Dose-response curve analysis by immunofluorescence

384-tissue culture plate was seeded with 1.2 × 10⁴ Vero cells per well. After 24 hours, Example 1, Comparative Example 1, and Comparative Example 2 were serially diluted 2-fold in DMSO to prepare 10 points, and cells were treated with 500 µg/ml as the highest concentration. About 1 hour after the treatment, cells were infected with SARS-CoV-2 (0.0125 MOI) in a BSL3 facility and incubated at 37 °C for 24 hours. After fixing the cells with 4% paraformaldehyde (PFA), permeabilization was performed. Thereafter, the cells were stained with the primary antibody, anti-SARS-CoV-2 nucleocapsid (N) and secondary antibodies, Alexa Fluor 488-conjugated goat anti-rabbit IgG and Hoechst 33342. The fluorescence images of infected cells were acquired using a large-capacity image analysis instrument, Operetta (Perkin Elmer).

### 4. Image analysis

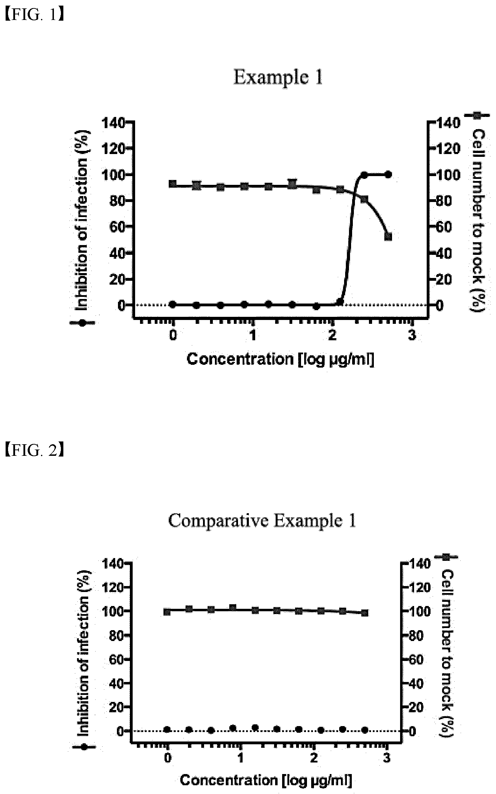

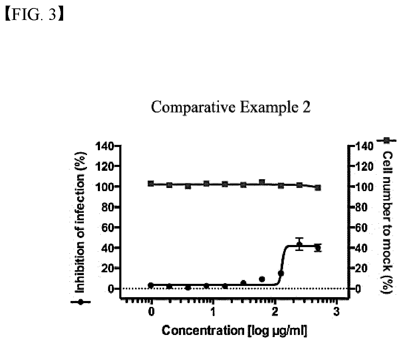

The fluorescence images of the cells obtained above were analyzed using Columbus software. The total number of cells per well was calculated as the number of Hoechst-stained nuclei, and the number of infected cells was calculated as the number of cells expressing the viral N protein. The infection ratio was calculated as the number of cells expressing the N protein/total number of cells. The infection ratio per well was normalized to the average infection ratio of wells containing uninfected cells (mock) and the average infection ratio of wells containing infected cells treated with 0.5% DMSO (v/v) in the same plate, and the resulting graphs are shown in FIGS. 1 to 3 (in the graphs of FIGS. 1 to 3, the circled line ( 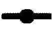 ) represents the inhibition of infection, and the squared line ( 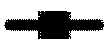 ) represents cytotoxicity). For cytotoxicity, the number of cells in each well was normalized to the average number of cells in the wells of the uninfected cell group and expressed as 'cell number to mock' on the graph. The response curve according to concentration and IC₅₀ and CC₅₀ values were derived using the formula Y = Bottom + (Top-Bottom)/(1 + (IC₅₀/X)^{Hillslope}) of XLFit 4 (IDBS) software. All IC₅₀ and CC₅₀ values were calculated from a fitted dose-response curve obtained through two repeated experiments, and selectivity index (SI) values were calculated as CC₅₀/IC₅₀ and are shown in Table 2.

**[Table 2]**

| | Example 1 | Comparative Example 1 | Comparative Example 2 |
|---|---|---|---|
| IC₅₀ | 36.08 *µ*g/ml | > 500 *µ*g/ml | > 500 *µ*g/ml |
| CC₅₀ | 92.2 *µ*g/ml | > 500 *µ*g/ml | > 500 *µ*g/ml |
| SI(Selectivity Index) | 2.56 | 1 | 1 |

As can be seen from the results of FIGS. 1 to 3 and Table 2, propolis (Comparative Example 1), which is known to be effective in suppressing Herpes Simplex Virus, and 1-Deoxynojirimycin (Comparative Example 2), which is known to be effective in suppressing severe acute respiratory syndrome virus (SARS-CoV), did not inhibit infection with severe acute respiratory syndrome virus-2 (SARS-CoV-2). On the other hand, Example 1 showed the SARS-CoV-2 infection inhibition rate of 96.24% at a concentration that does not show cytotoxicity, indicating that the camellia saponin according to an embodiment of the present invention was excellent in inhibiting coronavirus infection.

### [Formulation Example 1] Sanitizer

A sanitizer was prepared in a conventional manner according to the composition shown in Table 3 below.

**[Table 3]**

| Ingredients | Content(wt%) |
|---|---|
| Camellia saponin of Example 1 | 1.00 |
| Ethanol | 70 |
| Glycerin | 1.00 |
| Carbomer | 0.2 |
| Aminomethylpropanol | 0.1 |
| Purified water | balance |
| Total | 100.00 |

### [Formulation Example 2] Hand wash

A hand wash was prepared in a conventional manner according to the composition shown in Table 4 below.

**[Table 4]**

| Ingredient | Content(wt%) |
|---|---|
| Camellia saponin of Example 1 | 1.00 |
| Ethanol | 10 |
| Glycerin | 7.00 |
| Butylene glycol | 3.0 |
| Lauric acid | 3.0 |
| Lauryl hydroxysultaine | 2.4 |
| Myristic acid | 2.0 |
| Potassium hydroxide | 1.5 |
| Disodium EDTA | 0.1 |
| Purified water | balance |
| Total | 100.00 |

### [Formulation Example 3] Lotion

A lotion was prepared in a conventional manner according to the composition shown in Table 5 below.

**[Table 5]**

| Ingredient | Content(wt%) |
|---|---|
| Camellia saponin of Example 1 | 1.00 |
| L-ascorbic acid-2-magnesium phosphate salt | 1.00 |
| Collagen | 1.00 |
| Sodium citrate | 0.10 |
| Citric acid | 0.05 |
| Licorice extract | 0.20 |
| 1,3-butylene glycol | 3.00 |
| Purified water | balance |
| Total | 100.00 |

### [Formulation Example 4] Cream

A cream was prepared in a conventional manner according to the composition shown in Table 6 below.

**[Table 6]**

| Ingredient | Content(wt%) |
|---|---|
| Camellia saponin of Example 1 | 1.20 |
| Polyethylene glycol monostearate | 2.00 |
| Self-emulsifying glycerin monostearate | 5.00 |
| Cetyl alcohol | 4.00 |
| Squalene | 6.00 |
| Tri2-ethylhexane glyceryl | 6.00 |
| Sphingoglycolipid | 1.00 |
| 1,3-butylene glycol | 7.00 |
| Purified water | balance |
| Total | 100.00 |

### [Formulation Example 5] Soap

A soap was prepared in a conventional manner according to the composition shown in Table 7 below.

**[Table 7]**

| Ingredient | Content(wt%) |
|---|---|
| Camellia saponin of Example 1 | 1.00 |
| Titanium dioxide | 0.20 |
| Polyethylene glycol | 0.80 |
| Glycerin | 0.50 |
| Ethylenediaminetetraacetic acid | 0.05 |
| Sodium | 1.00 |
| Pigment | 0.20 |
| Soapy scent | 0.20 |
| Soap base | balance |
| Total | 100.00 |

### [Formulation Example 6] Pack

A pack was prepared in a conventional manner according to the composition shown in Table 8 below.

**[Table 8]**

| Ingredient | Content(wt%) |
|---|---|
| Camellia saponin of Example 1 | 1.20 |
| Polyvinyl alcohol | 13.00 |
| L-ascorbic acid-2-phosphate magnesium salt | 1.00 |
| Lauroylhydroxyproline | 1.00 |
| Collagen | 2.00 |
| 1,3-butylene glycol | 3.00 |
| Ethanol | 5.00 |
| Purified water | balance |
| Total | 100.00 |

### [Formulation Example 7] Cosmetic liquid formulation

A cosmetic liquid formulation was prepared in a conventional manner according to the composition shown in Table 9 below.

**[Table 9]**

| Ingredient | Content(wt%) |
|---|---|
| Camellia saponin of Example 1 | 1.00 |
| Hydroxyethylene cellulose | 12.00 |
| Xanthan gum | 2.00 |
| 1,3-butylene glycol | 6.00 |
| Glycerin | 4.00 |
| Sodium Hyaluronate | 5.00 |
| Purified water | balance |
| Total | 100.00 |

### [Formulation Example 8] Soft capsule

0.3 g of camellia saponin according to Example 1, 160 mg of L-carnitine, 320 mg of soybean oil, 2 mg of palm oil, 8 mg of hydrogenated vegetable oil, 4 mg of yellow beeswax and 6 mg of lecithin were mixed, and filled into one capsule according to a conventional method to prepare a soft capsule.

### [Formulation Example 9] Tablet

0.4 g of camellia saponin according to Example 1, 0.5 g of galactooligosaccharide, 80 mg of lactose and 220 mg of maltose were mixed and granulated using a fluidized bed dryer, and then 6 mg of sugar ester was added to the granulation, and the granulation was compressed with a tablet machine to prepare a tablet.

### [Formulation Example 10] Granules

0.4 g of camellia saponin according to Example 1, 250 mg of anhydrous crystalline glucose, and 550 mg of starch were mixed, formed into granules using a fluidized bed granulator, and then filled in a bag to prepare granules.

### [Formulation Example 11] Drink

0.4 g of camellia saponin according to Example 1, 10 g of glucose, 0.6 g of citric acid, and 25 g of liquid oligosaccharide are mixed, 500 ml of purified water was added to the mixture, and then the mixture was filled in a bottle. After that, the filled bottle was sterilized at 130 °C for 4 to 5 seconds to prepare a drink.

## Claims

1. A *camellia japonica* saponin for use in preventing, inhibiting or treating coronavirus infection, wherein the *camellia japonica* saponin is extracted from oil press cake of seeds of *camellia japonica,*
wherein the coronavirus is severe acute respiratory syndrome coronavirus-2 (SARS-CoV-2),
wherein the *camellia japonica* saponin comprises *camellia japonica* saponin A1, *camellia japonica* saponin A2, *camellia japonica* saponin B 1, *camellia japonica* saponin B2, *camellia japonica* saponin C1, or a combination thereof.

2. The *camellia japonica* saponin for its use according to claim 1, wherein the *camellia japonica* saponin is administered at a dose of 60 to 150 mg/kg/day.

3. The *camellia japonica* saponin for its use according to any one of claims 1 and 2, wherein the *camellia japonica* saponin is formulated in the form of a composition for skin external application.

4. The *camellia japonica* saponin for its use according to any one of claims 1 to 3, wherein the *camellia japonica* saponin is formulated in the form of a pharmaceutical composition.

## Patentansprüche

1. *Camellia japonica*-Saponin zur Verwendung beim Vorbeugen, Hemmen oder Behandeln einer Infektion mit Coronaviren, wobei das *Camellia japonica*-Saponin aus Ölpresskuchen von Samen der *Camellia japonica* extrahiert wird,
wobei das Coronavirus das schwere akute respiratorische Syndrom Coronavirus-2 (SARS-CoV-2) ist,
wobei das *Camellia japonica-*Saponin *Camellia japonica*-Saponin A1, *Camellia japonica*-Saponin A2, *Camellia japonica*-Saponin B1, *Camellia japonica-*Saponin B2, *Camellia japonica-*Saponin C1 oder eine Kombination davon umfasst.

2. *Camellia japonica*-Saponin zu dessen Verwendung nach Anspruch 1, wobei das *Camellia japonica*-Saponin in einer Dosis von 60 bis 150 mg/kg/Tag verabreicht wird.

3. *Camellia japonica*-Saponin zu dessen Verwendung nach einem der Ansprüche 1 und 2, wobei das *Camellia japonica*-Saponin in Form einer Zusammensetzung zur äußerlichen Anwendung auf der Haut formuliert ist.

4. *Camellia japonica*-Saponin zu dessen Verwendung nach einem der Ansprüche 1 bis 3, wobei das *Camellia japonica*-Saponin in Form einer pharmazeutischen Zusammensetzung formuliert ist.

## Revendications

1. Saponine de *camellia japonica* pour une utilisation dans la prévention, l'inhibition ou le traitement d'une infection à coronavirus, où la saponine de *camellia japonica* est extraite d'un tourteau d'huile de graines de *camellia japonica,*
dans laquelle le coronavirus est le coronavirus du syndrome respiratoire aigu sévère 2 (SARS-CoV-2),
dans laquelle la saponine de *camellia japonica* comprend la saponine A1 de *camellia japonica,* la saponine A2 de *camellia japonica,* la saponine B1 de *camellia japonica,* la saponine B2 de *camellia japonica,* la saponine C1 de *camellia japonica,* ou une combinaison de celles-ci.

2. Saponine de *camellia japonica* pour son utilisation selon la revendication 1, dans laquelle la saponine de *camellia japonica* est administrée à une dose allant de 60 à 150 mg/kg/jour.

3. Saponine de *camellia japonica* pour son utilisation selon l'une quelconque des revendications 1 et 2, dans laquelle la saponine de *camellia japonica* est formulée sous la forme d'une composition pour une application cutanée externe.

4. Saponine de *camellia japonica* pour son utilisation selon l'une quelconque des revendications 1 à 3, dans laquelle la saponine de *camellia japonica* est formulée sous la forme d'une composition pharmaceutique.
